# FASCICULE DE BREVET EUROPEEN

(11) **EP 1 720 495 B1**
(45) Date de publication et mention de la délivrance du brevet: **20.04.2011**
(21) Numéro de dépôt: 05729308.6
(22) Date de dépôt: 18.02.2005
(51) Int. Cl.: A61F 5/00

(54) **BALLON INTRA-GASTRIQUE AVEC MOYENS DE MISE EN FORME AMELIORES ET A RESISTANCE MECANIQUE RENFORCEE**
MAGENBALLON MIT VERBESSERTEM FORMMITTEL UND ERHÖHTER MECHANISCHER FESTIGKETI
INTRAGASTRIC BALLOON WITH IMPROVED FORMING MEANS AND INCREASED MECHANICAL STRENGTH

(30) Priorité: 20.02.2004 FR 0401733
(43) Date de publication de la demande: 15.11.2006
(73) Titulaire: Compagnie Européenne d'Etude et de Recherche de Dispositifs pour l'Implantation par Laparoscopie, 38200 Vienne (FR)
(72) Inventeur: PAGANON, Pascal, 69360 Serezin du Rhône (FR); RICOL, Jean-Paul, Gilbert, F-69210 Saint Germain sur L' Abresle (FR)
(74) Mandataire: Croonenbroek, Thomas Jakob
(86) Numéro de dépôt international: PCT/FR2005/000389
(87) Numéro de publication internationale: WO 2005/089686

(56) Documents cités:
- FR-A- 2 834 202
- US-A- 4 739 758
- US-A- 5 084 061

## Description

### DOMAINE TECHNIQUE

La présente invention se rapporte au domaine technique général des dispositifs implantables dans le corps humain destinés à être utilisés dans le cadre d'un traitement de l'obésité, et notamment de l'obésité morbide, et tout particulièrement à des implants aptes à réduire artificiellement le volume de l'estomac en vue notamment de produire une sensation de satiété chez le patient.

La présente invention se rapporte à un ballon intra-gastrique expansible destiné à être implanté dans l'estomac d'un patient dans le cadre d'un traitement de l'obésité, et comportant une enveloppe externe suffisamment souple pour passer d'une configuration à volume réduit à une configuration expansée conférant au ballon sa forme fonctionnelle.

### TECHNIQUE ANTERIEURE

Les ballons intra-gastriques utilisés pour le traitement de l'obésité se présentent généralement sous la forme d'une poche souple, formant l'enveloppe externe du ballon, et susceptible d'être remplie, une fois le ballon implanté à l'intérieur de l'estomac, avec un fluide de gonflage, notamment de l'air, pour conférer au ballon sa forme fonctionnelle, c'est-à-dire un volume et une forme d'utilisation qui lui permettent d'occuper une importante partie de l'espace disponible pour les aliments. La demande française FR-2834202 présente un exemple de ballon intra-gastrique composé de différentes poches, chacune étant susceptible d'être remplie par un fluide de gonflage.

Bien qu'ils présentent de multiples avantages, notamment liés à leur facilité de fabrication, de tels ballons souffrent néanmoins de plusieurs inconvénients.

En particulier, les ballons intra-gastriques connus présentent souvent une certaine porosité, laquelle va permettre la fuite progressive du fluide contenu dans le ballon (gaz ou liquide), et entraîner la diminution progressive du volume du ballon, et donc l'efficacité thérapeutique de ce dernier.

Ainsi, lorsque le volume du ballon ne correspond plus au volume souhaité pour le traitement de l'obésité, ce dernier ne crée plus la sensation de satiété voulue chez le patient.

Ce problème s'avère particulièrement critique dans les cas où l'enveloppe externe du ballon est uniquement remplie de gaz.

Dans certains cas extrêmes, la diminution du volume du ballon peut entraîner le passage de ce dernier à travers le pylore dans les voies intestinales, ce qui peut provoquer des complications sévères chez le patient.

Les propriétés ou caractéristiques du ballon intra-gastrique, telles que son étanchéité, responsables du maintien du ballon dans sa configuration fonctionnelle au cours du temps, apparaissent donc comme des paramètres essentiels dont la maîtrise permet d'améliorer l'efficacité du traitement thérapeutique.

On voit donc tout l'intérêt de réaliser un ballon adapté pour conserver son volume et sa configuration fonctionnelle dans le temps, et ce même lorsque l'enveloppe externe, soumise aux agressions extérieures et notamment aux sucs digestifs, se détériore et devient progressivement poreuse.

En outre, les ballons intra-gastriques généralement rencontrés souffrent également d'une certaine fragilité, qui provient notamment du fait que la poche renfermant le gaz (ou le liquide) de gonflage forme également l'enveloppe externe du ballon. Ladite poche subit donc directement les agressions extérieures, ce qui augmente le risque de perforation, notamment lorsque le ballon est manipulé par le praticien.

On voit donc tout l'intérêt de réaliser un ballon intra-gastrique, qui, tout en étant suffisamment élastique, soit également suffisamment résistant et protégé mécaniquement des agressions extérieures.

### EXPOSE DE L'INVENTION

Les objets assignés à l'invention visent en conséquence à porter remède aux différents inconvénients énumérés précédemment et à proposer un nouveau ballon intra-gastrique expansible pour le traitement de l'obésité ne présentant pas les inconvénients énumérés précédemment et qui, une fois positionné au sein de l'estomac dans sa configuration fonctionnelle, présente de très faibles variations de volume au cours du temps.

Un autre objet de l'invention vise à proposer un nouveau ballon intra-gastrique dont l'étanchéité est améliorée, et dont les pertes de fluide de gonflage, notamment de gaz, sont limitées, augmentant ainsi la durée d'efficacité thérapeutique du ballon.

Un autre objet de l'invention vise à proposer un nouveau ballon intra-gastrique qui, tout en présentant de bonnes propriétés d'élasticité, soit suffisamment résistant et protégé mécaniquement des agressions extérieures.

Un autre objet de l'invention vise à proposer un nouveau ballon intra-gastrique dont la mise en forme et l'expansion sont particulièrement simplifiées et rapides.

Un autre objet de l'invention vise à proposer un nouveau ballon intra-gastrique qui, bien que présentant un volume non négligeable, soit suffisamment léger, atraumatique et bien supporté par le patient.

Les objets assignés à l'invention sont atteints à l'aide d'un ballon intra-gastrique expansible destiné à être implanté dans l'estomac d'un patient dans le cadre d'un traitement de l'obesité tel que décrit dans la revendication 1.

### DESCRIPTIF SOMMAIRE DES DESSINS

D'autres objets et avantages de l'invention apparaîtront mieux à la lecture de la description qui suit, ainsi qu'à l'aide des dessins annexés, donnés à titre purement illustratif et non limitatif, dans lesquels :
- La figure 1 illustre, selon une vue en coupe transversale, un ballon intra-gastrique conforme à l'invention dans sa position d'expansion maximale.
- La figure 2 illustre, selon une vue en coupe transversale, un ballon intra-gastrique conforme à l'invention dans sa position non expansée.

### MEILLEURE MANIERE DE REALISER L'INVENTION

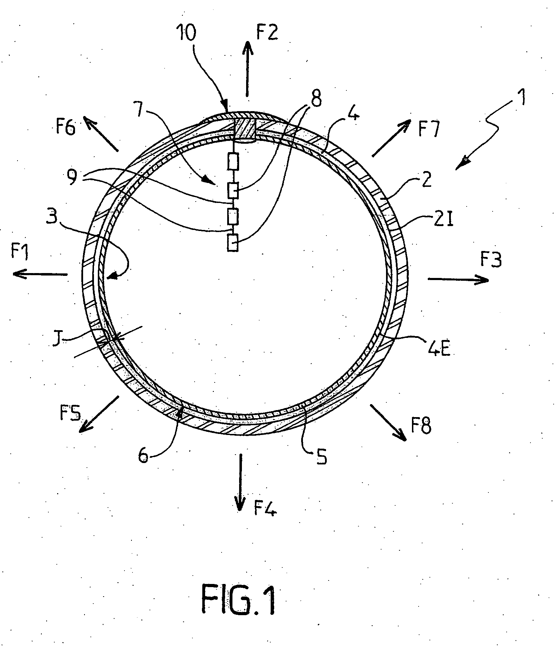

Le ballon intra-gastrique conforme à l'invention va maintenant être décrit en se référant aux figures 1 et 2.

Le ballon 1 intra-gastrique conforme à l'invention est destiné à être implanté dans l'estomac d'un patient dans le cadre d'un traitement de l'obésité.

Le ballon 1 intra-gastrique est expansible et comporte à cet effet une enveloppe externe 2 suffisamment souple pour passer d'une configuration à volume réduit (illustrée sur la figure 2) à une configuration expansée (illustrée sur la figure 1).

La configuration à volume réduit peut par exemple correspondre à une configuration dans laquelle le ballon 1 se présente dans une position repliée occupant un volume réduit, facilitant l'introduction du ballon 1 dans l'oesophage.

L'implantation du ballon 1 intra-gastrique conforme à l'invention est en effet généralement réalisée, de manière classique et bien connue de l'homme du métier, par passage dans les voies orales oesophagiennes sous sa forme repliée, comprimée ou lâche. L'expansion, la mise en place et la libération du ballon interviennent à la fin de l'opération gastro-endoscopique, lorsque le ballon 1 intra-gastrique est correctement positionné dans l'estomac du patient.

Ainsi, c'est dans sa configuration expansée que le ballon 1 va pouvoir occuper un volume non négligeable dans la cavité gastrique (non représentée), la configuration dite expansée conférant alors au ballon sa forme fonctionnelle.

L'expression « *forme fonctionnelle* » fait ainsi référence d'une part au volume, et d'autre part à la forme d'utilisation thérapeutique du ballon dans le cadre d'un traitement de l'obésité.

Ainsi, en occupant une partie du volume interne de l'estomac, le ballon 1 intra-gastrique selon l'invention permet de créer chez le patient une sensation rapide de satiété, qui s'accompagne d'une diminution de la quantité d'aliments ingérés.

Selon l'invention, le ballon 1 intra-gastrique comporte des moyens de mise en forme 3 de l'enveloppe externe 2.

Ces moyens de mise en forme 3 sont intégrés structurellement au ballon, c'est-à-dire qu'ils font partie du ballon avant même que ce dernier ne soit implanté au sein de la cavité gastrique. Plus précisément, les moyens de mise en forme 3 sont intégrés au ballon 1 lors de sa fabrication, formant ainsi une partie des composants fonctionnels du ballon.

Selon l'invention, les moyens de mise en forme 3 de l'enveloppe externe 2 sont susceptibles, une fois le ballon 1 implanté ou mis en place au sein de l'estomac, d'être actionnés pour :
- d'une part exercer une pression motrice suffisante sur l'enveloppe externe 2 pour la contraindre à se déployer,
- et d'autre part occuper, au sein de ladite enveloppe externe 2, un volume suffisant pour assurer le déploiement de l'enveloppe externe 2 de sa configuration à volume réduit jusqu'à sa configuration expansée.

Ainsi, les moyens de mise en forme 3 sont dimensionnés pour exercer une contrainte sur l'enveloppe externe 2 d'intensité sensiblement supérieure à la force de résistance susceptible d'être opposée par ladite enveloppe externe 2.

Au sens de l'invention, l'enveloppe externe 2 désigne la paroi extérieure du ballon intra-gastrique 1, délimitée intérieurement par une surface interne 2I et extérieurement par une surface externe 2 E formant la surface de contact du ballon 1 avec l'estomac.

Les moyens de mise en forme 3 sont configurés et adaptés pour exercer une contrainte sensiblement normale et centrifuge sur l'enveloppe externe 2, de manière à repousser cette dernière suivant les flèches F1 à F6 représentées sur la figure 1 d'une telle façon que la contrainte s'exerce sur sensiblement toute la surface interne 2I de l'enveloppe externe 2, c'est-à-dire sensiblement selon toutes les directions de l'espace.

Les moyens de mise en forme 3 sont indépendants de l'enveloppe externe 2, et adaptés pour occuper un volume croissant au sein de l'enveloppe externe 2 de manière à générer le déploiement de cette dernière. Plus précisément, les moyens de mise en forme 3 sont majoritairement disjoints de l'enveloppe externe 2, de telle sorte que lorsque le ballon 1 se trouve dans une configuration non expansée ou pliée, illustrée sur la figure 2, les moyens de mise en forme 3 ne sont pas nécessairement en contact avec l'enveloppe externe 2 sur toute la surface interne 2I de cette dernière.

Les moyens de mise en forme 3 sont ainsi montés au sein de l'enveloppe externe 2 avec une possibilité de mobilité relativement à cette dernière.

Les moyens de mise en forme 3 sont ainsi adaptés pour occuper un volume suffisant au sein de l'enveloppe externe 2 de telle sorte que cette dernière puisse atteindre sa configuration expansée.

De façon préférentielle, les moyens de mise en forme 3 présentent des propriétés de souplesse et d'élasticité, particulièrement intéressantes dans le présent domaine d'application.

La conception du ballon 1 conforme à l'invention, et notamment le recours aux moyens de mise en forme 3 décrits ci-dessus, permet ainsi d'obtenir un ballon 1 intra-gastrique susceptible de conserver sa forme fonctionnelle au cours du temps alors même que l'enveloppe externe 2 se détériore progressivement.

Selon une première variante possible de l'invention, non représentée aux figures, les moyens de mise en forme peuvent être avantageusement formés par une mousse visco-élastique à mémoire de forme, présentant des propriétés de rappel élastique en direction centrifuge suffisantes pour déployer l'enveloppe externe du ballon, lorsque cette dernière se trouve dans une configuration initiale à volume réduit au sein de l'estomac.

Dans cette première variante, on pourra réaliser, par exemple à l'aide d'un cathéter, une communication fluidique entre l'intérieur de l'enveloppe externe et l'atmosphère pour que la mousse puisse se détendre tout en contraignant l'enveloppe externe à se déployer, de l'air étant alors progressivement aspiré dans l'enveloppe en raison de l'expansion de la mousse.

Selon l'invention, représentée sur la figure 1, les moyens de mise en forme 3 sont formés par une chambre de gonflage 4 différente de l'enveloppe externe 2, et disposée à l'intérieur de cette dernière de manière à assurer la mise en forme de ladite enveloppe externe 2 par introduction d'un fluide de gonflage au sein de ladite de chambre de gonflage. 4.

L'expression « *chambre de gonflage* » désigne ici un organe gonflable, monté à l'intérieur de l'enveloppe externe 2, et délimitant un volume intérieur susceptible d'être rempli avec un fluide de gonflage.

La chambre de gonflage 4 et l'enveloppe externe 2 sont majoritairement disjointes, et sont reliées ou solidarisées au niveau d'un moyen de connexion 10, du genre valve, permettant la connexion de la chambre de gonflage 4 à un réservoir de fluide, pour effectuer le remplissage de la chambre de gonflage 4.

La chambre de gonflage 4 est ainsi susceptible d'être actionnée par gonflage à l'aide d'un fluide tel qu'un gaz (ou encore un liquide). Au fur et à mesure de son gonflage, la chambre de gonflage 4 va ainsi s'expanser et exercer une contrainte sur l'enveloppe externe 2, précisément sur la surface interne 21 de cette dernière, tendant à la repousser en direction centrifuge, tel que cela est représenté sur la figure 1 par les flèches F1 à F6, et ce jusqu'à ce que l'enveloppe externe 2 atteigne sa configuration expansée et fonctionnelle.

La chambre de gonflage 4 et l'enveloppe externe 2 sont ainsi conformées de telle manière que lors de l'opération de gonflage, l'enveloppe externe 2 n'exerce qu'une faible résistance en regard de la pression exercée sur sa surface interne 2I par la chambre de gonflage 4.

La chambre de gonflage 4 constitue ainsi une barrière étanche entre le fluide de gonflage qu'elle contient et l'enveloppe externe 2, empêchant le fluide de gonflage de migrer vers l'enveloppe externe 2 du ballon 1, dont l'étanchéité est plus faible que la chambre de gonflage 4.

En outre, l'enveloppe externe 2 étant séparée et indépendante des moyens de mise en forme 3, notamment de la chambre de gonflage 4, elle est susceptible d'assurer de manière efficace la protection mécanique de ces derniers.

A cet effet, l'enveloppe externe 2 sera préférentiellement réalisée à partir de matériaux souples, élastiques et présentant de bonnes propriétés de résistance mécanique.

La chambre de gonflage 4 et l'enveloppe externe 2 sont conformées pour que, lorsque la chambre de gonflage 4 occupe sa position expansée, l'enveloppe externe 2 vienne sensiblement épouser la forme de ladite chambre de gonflage 4.

Dans cette position dite expansée, la chambre de gonflage 4 pourra être surgonflée, c'est-à-dire remplie d'une quantité de fluide sensiblement supérieure à la quantité de fluide qu'elle est normalement capable de contenir sans subir de déformation ou encore d'extension élastique de sa paroi.

De façon préférentielle, la chambre de gonflage 4 et l'enveloppe externe 2 sont conformées pour que lors de l'opération de gonflage, un léger jeu subsiste entre la surface externe 4E de la chambre de gonflage et la surface interne 2I de l'enveloppe externe 2, ce jeu J étant suffisant pour autoriser une certaine mobilité relative entre la chambre de gonflage 4 et l'enveloppe externe 2.

Une telle mobilité permet ainsi d'une part d'éviter que l'enveloppe externe 2 n'entrave le gonflage de la chambre de gonflage 4, et d'autre part d'empêcher la formation de plis sur l'enveloppe externe 2. La chambre de gonflage 4 est formée par une poche interne 5 suffisamment souple pour passer d'une position à volume réduit à une position expansée.

La poche interne 5 est préférentiellement formée par un matériau élastomère, présentant une élasticité suffisante pour autoriser un surgonflage de ladite poche interne 5.

La chambre de gonflage 4, précisément la poche interne 5, est destinée à être remplie avec un gaz formant le fluide de gonflage. Bien évidemment, le fluide de gonflage pourrait être un liquide et ce, sans sortir du cadre de l'invention.

Le ballon intra-gastrique 1 forme ainsi, avec la chambre de gonflage 4 (ou poche interne 5) et l'enveloppe externe 2, constituant une poche externe, un ballon double poches.

Avantageusement, la poche interne 5 est délimitée par une paroi 6, distincte et majoritairement dissociée de l'enveloppe externe 2, comprenant au moins un écran sensiblement imperméable aux gaz, afin d'améliorer l'étanchéité globale du ballon. La paroi 6 peut ainsi avantageusement être formée par un film multi-couches, l'écran formant alors l'une des couches du film, ou par un film mono-couche, auquel cas l'écran forme l'unique couche du film.

De façon particulièrement avantageuse, l'écran comprend dans sa composition au moins un polymère à effet barrière aux gaz.

Par « *polymère à effet barrière aux gaz* », on désigne des polymères présentant une perméabilité aux gaz nettement inférieure à celle du silicone, et notamment une perméabilité à l'oxygène (O₂) inférieure à environ 10 fois celle du silicone.

L'écran pourra ainsi être composé d'un ou plusieurs polymères thermoplastiques à effet barrière aux gaz, tels que l'éthylène vinyle alcool (EVOH), le polychlorure de vinylidène (PVDC), le poly-acrylonitrile (PAN), le polyamide (PA), le polyamide bi-orienté, le polyéthylène téréphtalate (PET), le polyéthylène téréphtalate bi-orienté, et le polyuréthane thermoplastique élastomère.

Selon une variante préférentielle de l'invention, la poche interne 5 est formée par un ou plusieurs film(s), par exemple deux films de polyuréthane thermoplastique élastomère, lesdits films pouvant être solidarisés ensemble, par exemple par collage, ou mobiles les uns par rapport aux autres.

Le polyuréthane présente, entre autres propriétés intéressantes, un faible effet collant, facilitant le pliage, et également des propriétés de mémoire de forme.

A titre d'exemple illustratif, la perméabilité à l'oxygène (O₂) du polyuréthane est de l'ordre de 350 cm³.60 µm / m². 24h . bar à 1600 cm³.100 µm.m².24h.1bar, alors que la perméabilité du silicone est de l'ordre de 130 000 cm³. 100 µm / m². 24h . bar (mesures effectuées à 37°C et 90% d'humidité relative).

Un tel matériau présente également des propriétés élastiques autorisant un surgonflage de la poche interne 5, tout en offrant une résistance mécanique satisfaisante.

Avantageusement, la chambre de gonflage 4 est adaptée pour présenter, dans sa position expansée, une forme sensiblement sphérique.

A cet effet, la poche interne 5 est préférentiellement fabriquée à l'aide d'un procédé de thermoscellage, thermosoudage ou encore de thermoformage. Plus précisément, le procédé de fabrication de la poche interne 5 comporte une étape d'assemblage, au cours de laquelle on assemble, par soudure ou collage, le long d'une ligne de soudure périphérique, une ou plusieurs feuilles (par exemple deux feuilles) de forme prédéterminée en matériau sensiblement imperméable aux gaz, tel que le polyuréthane thermoplastique élastomère, préalablement mises en forme, par exemple par thermoformage, de manière à leur conférer une forme hémisphérique.

Chaque feuille peut être formée par un unique film de polyuréthane, mais est de préférence formée par plusieurs, et par exemple, deux films de polyuréthane superposés, lesdits films pouvant être solidarisés ensemble, par exemple par collage, ou mobiles les uns par rapport aux autres.

L'enveloppe externe 2 est quant à elle préférentiellement réalisée à l'aide d'un procédé de moulage par injection.

De façon particulièrement avantageuse, la chambre de gonflage 4 et l'enveloppe externe 2 sont sensiblement concentriques et sphériques, la forme sphérique de l'enveloppe externe 2 conférant ainsi au ballon 1 intra-gastrique un caractère atraumatique.

Selon une caractéristique particulièrement avantageuse de l'invention, l'enveloppe externe est formée par un matériau bio-compatible susceptible d'être bien supporté par le patient.

Ainsi, l'enveloppe externe 2 pourra être formée par un matériau élastomère, du genre silicone, un tel matériau présentant notamment des propriétés élastiques et de résistance mécanique intéressantes.

L'enveloppe externe 2 présente avantageusement des propriétés d'opacité radiologique.

En outre, selon une variante préférentielle de l'invention, le silicone utilisé pour former l'enveloppe externe 2 est avantageusement coloré en blanc par traitement au sulfate de baryum.

De façon préférentielle, l'épaisseur de la poche interne 5 sera inférieure à l'épaisseur de l'enveloppe externe 2, cette dernière assurant alors une fonction de protection mécanique relativement à la poche interne 5, limitant les risques de détérioration et/ou de perforation de ladite poche interne 5.

Ainsi, la conception du ballon 1 selon l'invention permet d'avoir recours à deux matériaux différents pour réaliser la poche interne 5 et l'enveloppe externe 2, et également de réaliser une poche interne 5 d'épaisseur particulièrement faible, ce qui permet notamment de limiter l'espace occupé par le ballon 1 lorsqu'il se trouve dans sa configuration à volume réduit pour être implanté dans l'estomac d'un patient.

De façon particulièrement avantageuse, et pour améliorer encore davantage l'étanchéité globale du ballon, il est possible de recouvrir l'enveloppe externe 2 de parylène. De même, la chambre de gonflage 4 pourra également être recouverte de parylène.

Une telle mesure présente également l'avantage de réduire l'effet collant du silicone.

Selon une variante encore plus préférentielle de l'invention, le ballon 1 intra-gastrique comporte des moyens de lestage formés par des corps solides et denses 8, de préférence en tungstène, destinés à alourdir sensiblement le ballon 1.

En effet, lorsque la chambre de gonflage 4 est uniquement remplie de gaz, le ballon 1 peut avoir tendance à remonter dans la partie haute de l'estomac et gêner la pénétration des aliments dans la cavité gastrique.

Le recours aux moyens de lestage permet ainsi d'améliorer le positionnement du ballon 1 au sein de l'estomac et d'éliminer en même temps une source importante d'inconfort pour le patient.

De façon particulièrement avantageuse, les corps solides et denses 8 sont reliés les uns aux autres par des portions de fils 9, de manière à former une chaîne, laquelle est avantageusement disposée à l'intérieur de la chambre de gonflage 4 et attachée de préférence à la valve 10.

La mise en place terminale du ballon 1 intra-gastrique conforme à l'invention va maintenant être décrite.

Avant son implantation, le ballon 1 intra-gastrique conforme à l'invention se présente de préférence sous une forme repliée, voire comprimée, de manière à faciliter son introduction et son passage dans les voies orales du patient, et en particulier l'oesophage.

Le ballon 1 peut avantageusement être replié au sein d'une housse de maintien, destinée à faciliter l'implantation du ballon. Dans ce cas, le ballon est introduit, avec sa housse, au sein de la cavité gastrique puis libéré, et la housse est retirée.

Une fois positionné à l'intérieur de l'estomac du patient, le ballon 1 intra-gastrique doit subir plusieurs opérations de la part du praticien afin de le rendre fonctionnel, c'est-à-dire de lui donner un volume suffisant pour qu'il occupe une partie de l'espace de la cavité gastrique réservée aux aliments. Dans le cas du ballon représenté sur la figure 1, le praticien connecte, par exemple à l'aide d'un cathéter prolongé par une aiguille de gonflage, la valve 10 à une source de fluide, préférentiellement de l'air, pour gonfler la chambre de gonflage 4. Cette opération a pour effet de permettre l'expansion de ladite chambre de gonflage 4, laquelle va venir exercer une contrainte sur l'enveloppe externe 2 tendant à la repousser progressivement en direction centrifuge suivant les flèches F1 à F6.

L'opération de gonflage se poursuit ainsi jusqu'à ce que l'enveloppe externe 2 atteigne sa configuration expansée et fonctionnelle.

Lorsque cette configuration est atteinte, le praticien peut retirer du corps du patient tout le matériel nécessaire au gonflage, à savoir le cathéter et l'aiguille de gonflage, cette opération ayant pour effet de libérer le ballon.

Le système formé par l'association de la chambre de gonflage 4 avec l'enveloppe externe 2 présente ainsi plusieurs caractéristiques communes avec le couple classique chambre à air-pneu, notamment en termes de souplesse et d'élasticité mais également en termes de résistance mécanique et de protection du système vis-à-vis des agressions extérieures.

Le ballon 1 intra-gastrique selon l'invention est donc avantageusement conçu de manière à conserver sa forme fonctionnelle et son volume au cours du temps et ce, alors même que l'enveloppe externe 2 pourrait se détériorer progressivement.

Un autre avantage du ballon intra-gastrique conforme à l'invention est qu'il présente des propriétés de résistance mécanique particulièrement intéressantes, notamment grâce à l'enveloppe externe 2 formant un moyen de protection mécanique du ballon.

Un autre avantage du ballon 1 intra-gastrique conforme à l'invention est qu'il présente une étanchéité améliorée par rapport aux ballons connus.

Un autre avantage du ballon 1 intra-gastrique conforme à l'invention est qu'il est suffisamment robuste pour permettre une manipulation aisée par le praticien.

### POSSIBILITE D'APPLICATION INDUSTRIELLE

L'invention trouve son application industrielle dans la conception et la fabrication de dispositifs implantables de lutte contre l'obésité.

## Revendications

1. Ballon intra-gastrique expansible destiné à être implanté dans l'estomac d'un patient dans le cadre d'un traitement de l'obésité et comportant une enveloppe externe (2) suffisamment souple pour passer d'une configuration à volume réduit à une configuration expansée conférant au ballon (1) sa forme fonctionnelle, le ballon comportant aussi des moyens de mise en forme (3) de l'enveloppe externe (2), ces moyens (3) de mise en forme étant indépendants et séparés de l'enveloppe externe (2) et intégrés structurellement au ballon (1) et majoritairement disjoints de l'enveloppe externe (2), lesdits moyens de mise en forme (3) étant susceptibles, une fois le ballon (1) implanté, d'être actionnés pour d'une part exercer une pression motrice suffisante sur l'enveloppe externe (2) pour la contraindre à se déployer, et d'autre part occuper, au sein de ladite enveloppe externe (2), un volume suffisant pour assurer le déploiement de l'enveloppe externe (2) de sa configuration à volume réduit jusqu'à sa configuration expansée, **caractérisé en ce que** les moyens de mise en forme (3) sont formés par une chambre de gonflage (4), différente de l'enveloppe externe (2) et disposée à l'intérieur de cette dernière de manière à assurer sa mise en forme par introduction d'un fluide de gonflage au sein de ladite chambre de gonflage (4) et que ladite chambre de gonflage (4) et l'enveloppe externe (2) sont conformées pour que lorsque la chambre de gonflage (4) occupe sa position expansée, l'enveloppe externe (2) vienne sensiblement épouser la forme de ladite chambre de gonflage (4).

2. Ballon intra-gastrique selon la revendication 1 **caractérisé en ce que** la chambre de gonflage (4) est formée par une poche interne (5) suffisamment souple pour passer d'une position à volume réduit à une position expansée.

3. Ballon intra-gastrique selon la revendication 2 **caractérisé en ce que** la poche interne (5) est formée par un matériau élastomère.

4. Ballon intra-gastrique selon la revendication 2 ou 3 **caractérisé en ce que** la poche interne (5) est délimitée par une paroi (6) comprenant au moins un écran sensiblement imperméable aux gaz.

5. Ballon infra-gastrique selon la revendication 4 **caractérisé en ce que** l'écran comprend dans sa composition au moins un polymère à effet barrière aux gaz.

6. Ballon intra-gastrique selon la revendication 5 **caractérisé en ce que** l'écran est composé d'un ou plusieurs polymères thermoplastiques à effet barrière aux gaz.

7. Ballon intra-gastrique selon la revendication 6 **caractérisé en ce que** l'écran est composé d'un ou plusieurs polymères choisis dans le groupe des polymères à effet barrière aux gaz tels que l'éthylène vinyle alcool (EVOH), le polychlorure de vinylidène (PVDC), le poly-acrylonitrile (PAN), le polyamide (PA), le polyamide bi-orienté, le polyéthylène téréphtalate (PET), le polyéthylène téréphtalate bi-orienté, et le polyuréthane thermoplastique élastomère.

8. Ballon intra-gastrique selon l'une des revendications 2 à 7 **caractérisé en ce que** la poche interne (5) est formée par au moins un film de polyuréthane thermoplastique élastomère.

9. Ballon intra-gastrique selon l'une des revendications précédentes **caractérisé en ce que** l'enveloppe externe (2) est formée par un matériau biocompatible.

10. Ballon intra-gastrique selon l'une des revendications précédentes **caractérisé en ce que** l'enveloppe externe (2) est formée par un matériau élastomère.

11. Ballon intra-gastrique selon la revendication 10 **caractérisé en ce que** l'enveloppe externe (2) est en silicone.

12. Ballon intra-gastrique selon la revendication 11 **caractérisé en ce que** le silicone est coloré en blanc par traitement au sulfate de baryum.

13. Ballon intra-gastrique selon l'une des revendications précédentes **caractérisé en ce que** l'enveloppe externe (2) est recouverte de parylène.

14. Ballon intra-gastrique selon l'une des revendications 1 à 13 **caractérisé en ce que** le fluide de gonflage est un gaz.

15. Ballon intra-gastrique selon l'une des revendications 1 à 14 **caractérisé en ce que** la chambre de gonflage (4) est adaptée pour présenter, dans sa position expansée, une forme sensiblement sphérique.

16. Ballon intra-gastrique selon l'une des revendications 1 à 15 **caractérisé en ce que** la chambre de gonflage (4) et l'enveloppe externe (2) sont sensiblement mobiles l'une par rapport à l'autre.

17. Ballon intra-gastrique selon l'une des revendications 1 à 16 **caractérisé en ce que** la chambre de gonflage (4) et l'enveloppe externe (2) sont sensiblement concentriques.

18. Ballon intra-gastrique selon l'une des revendications 1 à 17 **caractérisé en ce que** la chambre de gonflage (4) est recouverte de parylène.

## Claims

1. Expandable intragastric balloon designed to be implanted in the stomach of a patient for the treatment of obesity and having an outer envelope (2) that is sufficiently flexible to change from a reduced-volume configuration to an expanded configuration in which the balloon (1) acquires its functional shape, this balloon also having means (3) for shaping the outer envelope (2), these shaping means (3) being independent of and separate from the outer envelope (2) and being structurally integrated in the balloon (1) and for the most part detached from the outer envelope (2), said shaping means (3) being able to be actuated, once the balloon (1) has been implanted, in order to exert a sufficient driving pressure on the outer envelope (2) to force the latter to deploy, and in order to occupy, within said outer envelope (2), a sufficient volume to ensure the deployment of the outer envelope (2) from the reduced-volume configuration thereof to the expanded configuration thereof, **characterized in that** the shaping means (3) are formed by an inflation chamber (4), which is different from the outer envelope (2) and is arranged inside the latter so as to ensure its shaping by introduction of an inflating fluid into said inflation chamber (4), and **in that** said inflation chamber (4) and the outer envelope (2) are designed in such a way that, when the inflation chamber (4) occupies its expanded position, the outer envelope (2) substantially matches the shape of said inflation chamber (4).

2. Intragastric balloon according to Claim 1, **characterized in that** the inflation chamber (4) is formed by an inner pocket (5) that is sufficiently flexible to change from a reduced-volume position to an expanded position.

3. Intragastric balloon according to Claim 2, **characterized in that** the inner pocket (5) is formed by an elastomeric material.

4. Intragastric balloon according to Claim 2 or 3, **characterized in that** the inner pocket (5) is delimited by a wall (6) that comprises at least one shield substantially impermeable to gases.

5. Intragastric balloon according to Claim 4, **characterized in that** the shield comprises, in its composition, at least one polymer with a gas barrier effect.

6. Intragastric balloon according to Claim 5, **characterized in that** the shield is composed of one or more thermoplastic polymers with a gas barrier effect.

7. Intragastric balloon according to Claim 6, **characterized in that** the shield is composed of one or more polymers chosen from the group of polymers with a gas barrier effect, such as ethylene vinyl alcohol (EVOH), polyvinylidene chloride (PVDC), polyacrylonitrile (PAN), polyamide (PA), bi-oriented polyamide, polyethylene terephthalate (PET), bi-oriented polyethylene terephthalate, and elastomeric thermoplastic polyurethane.

8. Intragastric balloon according to one of Claims 2 to 7, **characterized in that** the inner pocket (5) is formed by at least one film of elastomeric thermoplastic polyurethane.

9. Intragastric balloon according to one of the preceding claims, **characterized in that** the outer envelope (2) is formed by a biocompatible material.

10. Intragastric balloon according to one of the preceding claims, **characterized in that** the outer envelope (2) is formed by an elastomeric material.

11. Intragastric balloon according to Claim 10, **characterized in that** the outer envelope (2) is made of silicone.

12. Intragastric balloon according to Claim 11, **characterized in that** the silicone is coloured white by treatment with barium sulphate.

13. Intragastric balloon according to one of the preceding claims, **characterized in that** the outer envelope (2) is covered with parylene.

14. Intragastric balloon according to one of Claims 1 to 13, **characterized in that** the inflating fluid is a gas.

15. Intragastric balloon according to one of Claims 1 to 14, **characterized in that** the inflation chamber (4) is designed to have a substantially spherical shape in its expanded position.

16. Intragastric balloon according to one of Claims 1 to 15, **characterized in that** the inflation chamber (4) and the outer envelope (2) are substantially movable in relation to each other.

17. Intragastric balloon according to one of Claims 1 to 16, **characterized in that** the inflation chamber (4) and the outer envelope (2) are substantially concentric.

18. Intragastric balloon according to one of Claims 1 to 17, **characterized in that** the inflation chamber (4) is covered with parylene.

## Patentansprüche

1. Expandierbarer intragastraler Ballon, der dazu vorgesehen ist, in den Magen eines Patienten im Rahmen einer Behandlung von Fettleibigkeit implantiert zu werden, und eine äußere Hülle (2) enthält, die ausreichend nachgiebig ist, um von einer Konfiguration mit verringertem Volumen in eine expandierte Konfiguration überzugehen, die dem Ballon (1) seine funktionale Form verleiht, wobei der Ballon außerdem Mittel (3) zum Formen der äußeren Hülle (2) enthält, wobei diese Formgebungsmittel (3) von der äußeren Hülle (2) unabhängig und getrennt sind und strukturell in den Ballon (1) integriert sind und von der äußeren Hülle (2) hauptsächlich getrennt sind, wobei die Formgebungsmittel (3), sobald der Ballon (1) implantiert ist, betätigt werden können, um einerseits auf die äußere Hülle (2) einen ausreichenden Steuerdruck auszuüben, um sie zu einem Entfalten zu drängen, und um andererseits innerhalb der äußeren Hülle (2) ein Volumen einzunehmen, das ausreicht, um das Entfalten der äußeren Hülle (2) aus ihrer Konfiguration mit verringertem Volumen in ihre expandierte Konfiguration sicherzustellen, **dadurch gekennzeichnet, dass** die Formgebungsmittel (3) durch eine von der äußeren Hülle (2) verschiedene Aufblaskammer (4) gebildet sind, die in dieser Letzteren in der Weise angeordnet sind, dass ihre Formung durch Einleiten eines Aufblasfluids in die Aufblaskammer (4) sichergestellt ist, und dass die Aufblaskammer (4) und die äußere Hülle (2) aneinander angepasst sind, damit sich die äußere Hülle (2) dann, wenn die Auf- blaskammer (4) ihre expandierte Position einnimmt, im Wesentlichen an die Form der Aufblaskammer (4) anschmiegt.

2. Intragastraler Ballon nach Anspruch 1, **dadurch gekennzeichnet, dass** die Aufblaskammer (4) durch eine Innentasche (5) gebildet ist, die ausreichend nachgiebig ist, um aus einer Position mit verringertem Volumen in eine expandierte Position überzugehen.

3. Intragastraler Ballon nach Anspruch 2, **dadurch gekennzeichnet, dass** die Innentasche (5) aus einem Elastomermaterial gebildet ist.

4. Intragastraler Ballon nach Anspruch 2 oder 3, **dadurch gekennzeichnet, dass** die Innentasche (5) durch eine Wand (6) begrenzt ist, die wenigstens eine für Gase im Wesentlichen undurchlässige Schicht enthält.

5. Intragastraler Ballon nach Anspruch 4, **dadurch gekennzeichnet, dass** die Schicht in seiner Verbindung wenigstens ein Polymer mit Gassperrenwirkung enthält.

6. Intragastraler Ballon nach Anspruch 5, **dadurch gekennzeichnet, dass** die Schicht aus einem oder mehreren thermoplastischen Polymeren mit Gassperrenwirkung zusammengesetzt ist.

7. Intragastraler Ballon nach Anspruch 6, **dadurch gekennzeichnet, dass** die Schicht aus einem oder mehreren Polymeren gebildet ist, die aus der Gruppe von Polymeren mit Gassperrwirkung gewählt sind, etwa: Ethylenvinyl-Alkohol (EVOH), Polychlorid-Vinyliden (PVDC), Polyacrylonitril (PAN), Polyamid (PA), biorientiertes Polyamid, Polyethylen-Terephthalat (PET), biorientiertes Polyethylen-Terephthalat und elastomeres thermoplastisches Polyurethan.

8. Intragastraler Bal-lon nach einem der Ansprüche 2 bis 7, **dadurch gekennzeichnet, dass** die Innentasche (5) durch wenigstens eine dünne Schicht aus thermoplastischem elastomeren Polyurethan gebildet ist.

9. Intragastraler Ballon nach einem der vorhergehenden Ansprüche, **dadurch gekennzeichnet, dass** die äußere Hülle (2) aus einem biokompatiblen Material gebildet ist.

10. Intragastraler Ballon nach einem der vorhergehenden Ansprüche, **dadurch gekennzeichnet, dass** die äußere Hülle (2) aus einem elastomeren Material gebildet ist.

11. Intragastraler Ballon nach Anspruch 10, **dadurch gekennzeichnet, dass** die äußere Hülle (2) aus Silikon besteht.

12. Intragastraler Ballon nach Anspruch 11, **dadurch gekennzeichnet, dass** das Silikon durch Behandlung mit Bariumsulfat weiß gefärbt ist.

13. Intragastraler Ballon nach einem der vorhergehenden Ansprüche, **dadurch gekennzeichnet, dass** die äußere Hülle (2) mit Parylen beschichtet ist.

14. Intragastraler Ballon nach einem der Ansprüche 1 bis 13, **dadurch gekennzeichnet, dass** das Aufblasfluid ein Gas ist.

15. Intragastraler Ballon nach einem der Ansprüche 1 bis 14, **dadurch gekennzeichnet, dass** die Aufblaskammer (4) dazu ausgelegt ist, in ihrer expandierten Position eine im Wesentlichen kugelförmige Gestalt aufzuweisen.

16. Intragastraler Ballon nach einem der Ansprüche 1 bis 15, **dadurch gekennzeichnet, dass** die Aufblaskammer (4) und die äußere Hülle (2) relativ zueinander im Wesentlichen beweglich sind.

17. Intragastraler Ballon nach einem der Ansprüche 1 bis 16, **dadurch gekennzeichnet, dass** die Aufblaskammer (4) und die äußere Hülle (2) im Wesentlichen konzentrisch sind.

18. Intragastraler Ballon nach einem der Ansprüche 1 bis 17, **dadurch gekennzeichnet, dass** die Aufblaskammer (4) mit Parylen beschichtet ist.
